# FASCICULE DE BREVET EUROPEEN

(11) **EP 3 016 747 B1**
(45) Date de publication et mention de la délivrance du brevet: **10.06.2020**
(21) Numéro de dépôt: 14749909.9
(22) Date de dépôt: 04.07.2014
(51) Int. Cl.: B05B 1/06, B65D 83/30, B65D 83/20, A61L 9/01, A61Q 15/00, A61K 8/31, A61K 8/69, A61K 8/04, A61K 8/891, B05B 7/04

(54) **AÉROSOL DÉODORANT ANHYDRE ÉQUIPÉ D'UNE TÊTE DE DISTRIBUTION CREUSE**
ANYHDRISCHES DESODORIERENDES AEROSOL MIT EINEM HOHLEN AUSGABEKOPF
ANHYDROUS DEODORANT AEROSOL EQUIPPED WITH A HOLLOW DISPENSING HEAD

(30) Priorité: 04.07.2013 FR 1356570
(43) Date de publication de la demande: 11.05.2016
(73) Titulaire: L'Oréal, 75008 Paris (FR)
(72) Inventeur: ALBISETTI, Nicolas, F-95210 Saint Gratien (FR)
(74) Mandataire: L'Oreal
(86) Numéro de dépôt international: PCT/FR2014/051722
(87) Numéro de publication internationale: WO 2015/001271

(56) Documents cités:
- WO-A1-2012/075899
- US-A- 5 215 260
- US-A- 5 297 739

## Description

La présente invention concerne les récipients équipés de têtes de distribution utilisées pour distribuer un produit cosmétique, notamment le pulvériser.

L'invention concerne plus particulièrement mais non exclusivement les récipients pressurisés équipés de têtes de pulvérisation contenant des produits cosmétiques déodorants ou anti transpirants.

De nombreuses récipients équipes de têtes de pulvérisation ont été proposées, à un ou à plusieurs orifice(s) de distribution. De nombreux paramètres sont à considérer lors de la conception d'une tête de pulvérisation.

Tout d'abord, le spray généré doit présenter la granulométrie convenant à l'application. A cet égard, la taille des gouttelettes ne doit pas être trop petite ni trop grande.

Par ailleurs, le spray généré doit être délivré avec le débit requis et la tête de pulvérisation ne doit pas opposer une trop forte perte de charge à la circulation du produit.

La forme du spray doit également correspondre à l'application visée et permettre ainsi, selon les cas, de couvrir une surface plus ou moins étendue.

Enfin, la tête de pulvérisation doit être esthétiquement attractive pour le consommateur, et sa fabrication compatible avec les exigences de production à large échelle.

La demande EP 1 052 023 A1 divulgue une tête de pulvérisation comportant un orifice de distribution défini entre un obturateur ayant une partie tronconique et le corps de la tête. L'obturateur s'ouvre par déformation de la partie tronconique, sous la pression du produit lors de la distribution. Il se forme un spray conique et creux, au moins au voisinage de la tête.

La demande WO 2011/065413 divulgue divers agencements de têtes de pulvérisation dans lesquels un orifice de pulvérisation est défini entre une partie périphérique et une partie centrale, reliées entre elles par des ponts de matière.

L'invention vise à proposer une nouvelle tête de distribution qui convienne tout particulièrement à la pulvérisation d'un produit cosmétique déodorant ou anti transpirant.

L'invention vise aussi à proposer un nouveau récipient équipé d'une tête de pulvérisation qui produise un effet visuel totalement original par rapport à ce qui existe actuellement.

L'invention vise aussi à proposer un récipient équipé d'une tête de pulvérisation procurant une sensation à l'application distincte des sensations ressenties avec les dispositifs actuels.

En outre, la pulvérisation doit être efficace pour l'application visée.

En particulier, dans le domaine des déodorants ou des anti transpirants, on cherche des têtes de pulvérisation avec lesquelles il est possible d'atteindre, précisément, une cible donnée.

Il existe un besoin pour accroître la rapidité d'application d'un produit par pulvérisation sans pour autant augmenter les risques de bouchage liés au séchage du produit pulvérisé.

L'invention vise ainsi à perfectionner encore les dispositifs connus tout en permettant l'utilisation de techniques de fabrication dont le coût est compatible avec une diffusion à grande échelle.

L'invention a pour objet, selon un premier de ses aspects, un récipient équipé d'une tête de distribution, le récipient contenant ladite composition cosmétique à distribuer:
i. la tête de distribution comporte :
   - un corps comportant une couronne s'étendant selon un axe de distribution Z, le corps étant ouvert à ses deux extrémités axiales opposées,
   - une partie coopérante s'étendant selon un axe de distribution Z et étant ouverte à ses deux extrémités axiales opposées, définissant au moins partiellement au moins un orifice de distribution, la partie coopérante étant engagée sur la couronne, la section transversale de l'orifice de distribution étant comprise entre 0,05 mm² et 0,5 mm²,
   - ii. la composition cosmétique comprend, dans un milieu cosmétiquement acceptable,
      - au moins une phase huileuse comprenant une ou plusieurs huiles choisie(s) parmi les huiles siliconées, hydrocarbonées et fluorées,
      - au moins un actif cosmétique, notamment au moins un actif déodorant et/ou un actif anti-transpirant.

En particulier, l'orifice de distribution est de préférence défini entre le corps et la partie coopérante mais peut, en variante, être défini entièrement par la partie coopérante.

Grâce à l'invention, un passage est ménagé à travers la tête de distribution et plus particulièrement à travers le corps et la partie coopérante, permettant à une circulation d'air de s'établir à travers la tête lorsque le produit à distribuer est émis, ce qui peut s'avérer avantageux lorsque le produit est émis sous la forme d'un spray, en permettant à un courant d'air d'être créé à travers la tête pour accompagner l'écoulement du spray.

De plus, la tête de distribution présente un aspect qui tranche clairement avec l'esthétique habituelle des têtes de distribution connues, et qui s'avère particulièrement attractif pour le consommateur.

Par ailleurs, le passage à travers la tête peut être réalisé avec des dimensions suffisantes pour permettre, si cela est recherché, d'introduire le doigt ou une mèche de cheveux dans ce passage. Cela peut faciliter l'application d'un produit sur le doigt ou la mèche de cheveux.

L'invention peut également permettre, si on le souhaite, de faciliter la réalisation d'un orifice de distribution de section annulaire entre la partie coopérante et le corps, permettant la formation d'un spray creux. En variante, plusieurs orifices de distribution sont formés entre le corps et la partie coopérante, par exemple dans le but de distribuer le produit sous la forme de plusieurs sprays ou jets. Le nombre d'orifices de distribution peut notamment être supérieur ou égal à 10, mieux 20, encore mieux 30. Les orifices de distribution ont par exemple chacun une section transversale supérieure ou égale à 0,003 mm², mieux 0,006 mm² et sont de préférence espacés entre eux (mesure selon une droite entre barycentres des orifices) d'une distance de plus de 1 mm.

Dans une autre variante, plusieurs orifices de distribution sont formés entièrement dans la partie coopérante. Les orifices peuvent être construits de manière à ce que le jet sortant de chaque orifice soit tourbillonnaire, notamment grâce à au moins deux canaux tourbillonnaires orientés de façon tangentielle autour de l'axe de l'orifice. La partie coopérante peut avoir, en demi-section axiale, une forme de U. Le corps peut présenter deux jupes de montage concentriques entre lesquelles se fixe la partie coopérante. La couronne peut un ou plusieurs reliefs définissant avec la partie coopérante des canaux d'amenée, notamment tourbillonnaires, vers l'orifice de distribution.

Le corps peut définir un logement dans lequel est reçue la partie coopérante, laquelle est alors qualifiée de noyau.

Le ou les orifices de distribution peuvent être ouverts au repos. Par « au repos » il faut comprendre avant que la partie coopérante ne soit exposée à la pression du produit à distribuer. Ainsi, dans ce cas, le ou les orifices de distribution sont déjà formés et ouverts quand le produit est envoyé dans la tête pour être distribué. En variante, l'orifice de distribution se forme au moment de la distribution du produit, grâce par exemple à l'élasticité d'une portion au moins du corps ou de la partie coopérante, qui se déforme sous la pression du produit au moment de la distribution.

Grâce à l'invention, dans le cas d'une pulvérisation, le spray peut être émis avec un débit relativement important, si on le souhaite, tout en ayant une tête de pulvérisation de construction relativement simple et de fonctionnement fiable. En particulier, l'orifice de distribution peut être réalisé avec des dimensions bien définies. De plus, la tête de distribution peut présenter une esthétique attractive pour le consommateur.

Le corps peut présenter une première surface s'évasant vers l'extérieur, respectivement convergeant vers l'extérieur, et la partie coopérante présenter une deuxième surface, en regard de la première surface, divergeant vers l'extérieur, respectivement convergeant vers l'extérieur. La première surface peut être conique. La deuxième surface peut être conique, de même angle que la première surface ou d'angle supérieur ou inférieur.

Un angle différent résultant en un rétrécissement de l'espace peut conduire à une accélération du jet avant sa sortie, ce qui peut être intéressant dans le cadre d'un spray.

L'orifice de distribution peut être unique ou non et peut présenter une forme annulaire ou non. L'orifice de distribution peut présenter, dans le sens circonférentiel, une largeur constante. Le ou les orifices de distribution peuvent être définis entre deux surfaces de révolution, concentriques, par exemple cylindriques de révolution.

Le ou les orifices de distribution peuvent être à symétrie axiale, de préférence à symétrie de révolution, autour de l'axe de distribution notamment. L'axe de distribution est défini par la direction générale vers laquelle le produit est distribué par la tête.

La partie coopérante est de préférence rapportée, ce qui facilite sa fabrication et celle du corps. En variante, la partie coopérante est moulée d'une seule pièce avec le corps, notamment dans le cas de la distribution d'une mousse, l'orifice de distribution pouvant alors présenter une section plus grande que dans le cas de la pulvérisation d'un spray.

L'espace ménagé entre le corps et la partie coopérante est alimenté par au moins un canal d'amenée dont la section est de préférence supérieure à celle de l'orifice de distribution, ce qui facilite le remplissage de cet espace avant que le produit ne sorte par l'orifice de distribution.

Une chambre de répartition de produit peut avantageusement être ménagée, entre la partie coopérante et le corps, en amont de l'orifice de distribution. Cela peut faciliter l'émission d'un spray homogène, notamment.

Le canal d'amenée du produit peut déboucher dans cette chambre, laquelle présente de préférence une forme annulaire. Sa largeur, qui correspond à l'intervalle entre la partie coopérante et le corps, est de préférence supérieure à la largeur maximale du passage par lequel la chambre de répartition communique avec l'orifice de distribution.

L'un au moins du corps et de la partie coopérante, de préférence le corps, peut présenter au moins un relief de centrage de la partie coopérante relativement au corps, et de préférence au moins dix, mieux au moins vingt, encore mieux au moins quarante. Ces reliefs peuvent venir jusqu'au bord de la pièce dans laquelle ils sont réalisés de manière à générer une multitude d'orifices par lesquels sortent les jets de produit, les reliefs de centrage étant notamment orientés parallèlement à l'axe de distribution ou obliquement dans le même sens circonférentiel autour de l'axe, pouvant encore éventuellement définir entre eux des rétrécissements de section conduisant à une accélération du jet de produit. Ce ou ces reliefs sont de préférence situés en retrait de l'orifice de distribution lorsque l'on cherche à générer un spray sous forme d'un jet unique. Les reliefs peuvent être réalisés sur le corps, étant par exemple sous la forme de nervures axiales équiréparties sur toute la surface du corps en regard de la partie coopérante.

Les reliefs de centrage peuvent éventuellement assurer à eux seuls le maintien de la partie coopérante sur le corps. En variante, la partie coopérante est fixée sur le corps ailleurs qu'au niveau des reliefs de centrage, les reliefs de centrage pouvant dans ce cas avoir ou non une fonction de maintien de la partie coopérante sur le corps.

De préférence, la partie coopérante est fixe relativement au corps. En variante, la partie coopérante est fixée de façon réglable relativement au corps, pour par exemple permettre à l'utilisateur de régler la largeur de l'orifice de distribution ou fermer celui-ci en l'absence d'utilisation, par exemple en la vissant d'un quart de tour, ce vissage s'accompagnant d'un déplacement axial de la partie coopérante relativement au corps.

La partie coopérante peut être affleurante à l'extrémité avant du corps de manière à générer un spray d'axe sensiblement parallèle à l'axe de la partie coopérante.

La partie coopérante peut dépasser axialement de l'extrémité avant du corps d'une valeur comprise entre 0 et 1 mm, mieux entre 0 et 0,5 mm. Le spray peut alors être divergent vers l'axe de la partie coopérante.

La partie cooopérante peut être axialement en retrait de l'extrémité avant du corps d'une valeur comprise entre 0 et 1 mm, mieux entre 0 et 0,5 mm. Le spray peut alors être convergent vers l'axe de la partie coopérante.

Avantageusement, l'orifice de distribution est de section transversale triangulaire.

Plus avantageusement, la partie coopérante définit au moins partiellement une pluralité d'orifices de distribution, la somme des sections transversales des orifices de distribution étant comprise entre 0,05 mm² et 0,5 mm²,

Encore plus avantageusement, le nombre d'orifices de distribution est supérieur ou égal à 5, de préférence supérieur ou égal à 10.

De préférence, la section transversale des orifices de distribution étant comprise entre 0,03 mm² et 0,4 mm², et de préférence entre 0,05 mm² et 0,4 mm².

L'invention permet de réaliser facilement, si on le souhaite, un orifice de distribution de contour intérieur circulaire. Le diamètre intérieur du passage formé à travers la tête est par exemple supérieur ou égal à 10 mm, mieux à 15 mm, 20 ou 30 mm. Lorsque le passage n'est pas de section circulaire, le « diamètre intérieur » désigne celui du plus grand cercle inscrit dans ce passage.

La tête peut comporter au moins deux logements et deux parties coopérantes disposées dans les logements et définissant chacune avec le corps, au repos, un orifice de distribution selon l'invention. Les axes de distribution peuvent alors être parallèles ou non, sécants ou non, par exemple converger l'un vers l'autre.

L'orifice de distribution peut être d'axe, en demi-coupe axiale, convergent ou divergent par rapport à la direction de pulvérisation.

L'invention concerne aussi un dispositif de distribution d'une composition cosmétique, ledit dispositif comprenant une tête de distribution destinée à équiper un récipient contenant ladite composition cosmétique à distribuer, la tête de distribution comportant :
- un corps ouvert à ses deux extrémités axiales opposées,
- une partie coopérante ouverte à ses deux extrémités axiales opposées, définissant au moins partiellement un orifice de distribution, et
   la composition cosmétique comprenant, dans un milieu cosmétiquement acceptable,
   - au moins une phase huileuse comprenant une ou plusieurs huiles choisie(s) parmi les huiles siliconées, hydrocarbonées et fluorées,
   - au moins un actif cosmétique, notamment au moins un actif déodorant et/ou un actif anti-transpirant.

### Caractéristiques des orifices :

Forme triangulaire: pour la facilité de mise en œuvre
Longueur du canal: comprise entre 0.5 et 2 mm, actuellement à 1.5mm, permet de créer un spray individuel avec un cône réduit
Type de valve sélectionnée (Gicleur = 1x0.5, Restriction Interne = 0.8, PGA = 0.4). La surface totale des orifices de l'anneau a été choisie pour être proche de la surface de l'orifice du Gicleur (ex : 1x0.5 = 0.196 mm²). Nous obtenons donc des sections d'orifices très faibles pour 20 trous (environ 0.01 mm²), plus important pour 10 trous (environ 0.02 mm²) et pour 5 orifices (environ 0.04 mm²)
Adéquation Formules / Anneaux pour une surface totale proche de la surface du diffuseur ou Gicleur = petits orifices
Emulsion : 20 à 10 orifices et Anhydre : 10 à 2 orifices
Adéquation Formules / Anneaux pour une surface individuelle proche de la surface du diffuseur ou Gicleur = gros orifices
Emulsion et Anhydre : 20 à 2 orifices

### Variantes

Voir point 4, Ceci permet d'imaginer l'étendue des possibilités tant en termes de nombres d'orifices que de sections ou puissances des sprays. De même le taux de gaz peut être réévalué en fonction des besoins.

Dans le cas d'un débit gérer par le diffuseur, le gicleur de la valve peut être plus important.

### Répartition des orifices

Equidistant sur la périphérie de l'anneau
Equidistant entre eux sur une portion de l'anneau
Répartie en groupe équidistant (ex : X portions équidistantes (sur la périphérie ou sur une portion de l'anneau) composés de plusieurs orifices équidistants)
Il est possible de créer un anneau supportant complétement les orifices de diffusions qui peuvent être cylindrique.
pour pallier la limite du nombre d'orifices on peut imaginer créer des sprays plats.

### COMPOSITION COSMETIQUE

Par "actif anti-transpirant", on entend toute substance qui, à elle seule, a pour effet de diminuer ou limiter le flux sudoral.

Par « actif déodorant" toute substance capable de masquer, absorber, améliorer et/ou réduire l'odeur désagréable résultant de la décomposition de la sueur humaine par des bactéries.

Par « cosmétiquement acceptable », on entend compatible avec la peau et/ou ses phanères, qui présente une couleur, une odeur et un toucher agréables et qui ne génère pas d'inconforts inacceptables susceptibles de détourner la consommatrice d'utiliser cette composition.

### Actifs déodorants

Les compositions cosmétiques selon l'invention peuvent contenir un ou plusieurs actifs déodorants comme par exemple
- des agents bactériostatiques ou des agents bactéricides tels que le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,5,10-triénol (Farnesol) ; les sels d'ammonium quaternaires comme les sels de cetyltrimethylammonium, les sels de cétylpyridinium ; la chlorhexidine et les sels; le monocaprate de diglycérol, le monolaurate de diglycérol , monolaurate de glycérol ; les sels de polyhexaméthylène biguanide ;
- des sels de zinc comme le salicylate de zinc , le phénolsulfonate de zinc, le pyrrolidone carboxylate de zinc (plus communément appelé pidolate de zinc), le sulfate de zinc, le chlorure de zinc, le lactate de zinc, le gluconate de zinc, ricinoléate de zinc, glycinate de zinc, carbonate de zinc, citrate de zinc, chlorure de zinc, le laurate de zinc, l'oléate de zinc, l' orthophosphate de zinc, le stéarate de zinc, le tartrate de zinc, le lactate de zinc, l'acétate de zinc ou leurs mélanges ;
- des absorbeurs d'odeurs comme les zéolites, les cyclodextrines, les silicates d'oxyde métallique telles que celles décrites dans la demande US2005/063928 ; des particules d'oxyde métallique modifiées par un métal de transition telles que décrites dans les demandes US2005084464 et US2005084474, des aluminosilicates comme ceux décrits dans la demande EP1658863, des particules de dérivés de chitosan comme celles décrites dans le brevet US6916465 ;
- des substances bloquant les réactions enzymatiques responsables de la formation des composés odorants comme les inhibiteurs d'arylsulfatase, de 5-Lipoxygenase, d'aminocylase, de β-glucoronidase ;
et leurs mélanges.

Les actifs déodorants peuvent être présents dans la composition selon l'invention à raison de 0,01 à 10% en poids par rapport au poids total de la composition et de préférence à raison de 0,1 à 5% en poids.

### Actifs anti-transpirants

Les actifs anti-transpirants sont choisis de préférence parmi les sels d'aluminium et/ou de zirconium ; les complexes d'hydroxychlorure de zirconium et d'hydroxychlorure d'aluminium avec un acide aminé tels que ceux décrits dans le brevet US-3792068 communément connus sous l'appellation "ZAG complexes. De tels complexes sont généralement connus sous l'appellation ZAG (lorsque l'acide aminé est la Glycine). Les complexes ZAG présentent d'ordinaire un quotient Al/Zr allant d'environ 1,67 à 12,5 et un quotient Métal/Cl allant d'environ 0,73 à 1,93. Parmi ces produits on peut citer l'aluminium zirconium octachlorohydrex GLY, l'aluminium zirconium pentachlorohydrex GLY, l'aluminium zirconium tetrachlorohydrate GLY et l'aluminium zirconium trichlorohydrate-GLY.

Parmi les sels d'aluminium, on peut citer le chlorhydrate d'aluminium, l'aluminium chlorohydrex, l'aluminium chlorohydrex PEG, l'aluminium chlorohydrex PG, l'aluminium dichlorohydrate, l'aluminium dichlorohydrex PEG, l'aluminium dichlorohydrex PG, l'aluminium sesquichlorohydrate, l'aluminium sesquichlorohydrex PEG, l'aluminium sesquichlorohydrex PG, les sels d'alun, l'aluminium sulfate, l'aluminium zirconium octachlorohydrate, l'aluminium zirconium pentachlorohydrate, l'aluminium zirconium tetrachlorohydrate, l'aluminium zirconium trichlorohydrate et plus particulièrement l'hydroxychlorure d'aluminium commercialisé par la société REHEIS sous la dénomination REACH 301 ou par la société GUILINI CHEMIE sous la dénomination ALOXICOLL PF 40. Des sels d'aluminium et de zirconium sont par exemple celui commercialisé par la société REHEIS sous la dénomination REACH AZP-908-SUF.

On utilisera plus particulièrement le chlorhydrate d'aluminium sous forme activée ou non activée.

Les actifs anti-transpirants peuvent être présents dans la composition selon l'invention à raison de 0,001 à 30% en poids par rapport au poids total de la composition et de préférence à raison de 0,5 à 25% en poids.

La composition selon l'invention a de préférence un pH allant de 3 à 9 selon le support choisi.

### MILIEU ANHYDRE

Par "anhydre", on entend au sens de l'invention, une composition dont la teneur totale en eau est inférieure à 3% en poids par rapport au poids total de la composition, et de préférence dont la teneur en eau est inférieure à 1% en poids par rapport au poids total de la composition.

Le véhicule liquide anhydre cosmétiquement acceptable contient une phase huileuse qui peut contenir une ou plusieurs huiles volatiles ou non volatiles, siliconées, hydrocarbonées ou fluorées.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones D4, D5 ou D6.

Les huiles hydrocarbonées volatiles additionnelles peuvent être choisies parmi notamment les alcanes ramifiés en C₈-C₁₆ comme les isoalcanes (appelées aussi isoparaffines) en C₈-C₁₆, l'isododécane, l'isodécane, l'isohexadécane et par exemple les huiles vendues sous les noms commerciaux d'Isopars ou de Permethyls', les esters ramifiés en C₈-C₁₆ comme le néo pentanoate d'iso-hexyle et leurs mélanges ; on utilise de préférence l'isododécane.

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 245 Fluid » ; les cyclopolydiméthylsiloxanes (cyclométhicones) ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, les polyméthyl-phénylsiloxanes ;

Les huiles fluorées non volatiles peuvent être choisies parmi celles partiellement hydrocarbonées et/ou siliconées comme celles décrites dans le document 0 JP-A-2-295912.

Les huiles hydrocarbonées non volatiles peuvent être d'origine minérale, synthétique ou animale. Elles peuvent être choisies parmi les huiles de paraffine comme la vaseline, l'huile de vaseline, le perhydrosqualène, les polydécènes, l'isohexadecane, l'isododecane, le polyisobutène hydrogéné (ou isoporaffine hydrogénée) tel que l'huile de Parléam ; les esters gras de préférence ceux obtenus à partir d'un alcool à chaîne linéaire ou ramifiée, ayant de 1 à 17 atomes de carbone et d'un acide gras à chaîne linéaire ou ramifiée, ayant de 3 à 18 et de préférence de 12 à 17 atomes de carbone. De façon avantageuse, ledit ester est un ester saturé qui ne renferme aucun groupement éther ni hydroxyle (la quantité totale de carbone dans l'ester peut varier de 12 à 50 et de préférence de 20 à 50).

Comme esters gras, on peut citer par exemple le caprate/caprylate d'éthyl-2 hexyle (ou caprate/caprylate d'octyle), le laurate d'éthyle, le laurate de butyle, le laurate d'hexyle, le laurate d'isohexyle, le laurate d'isopropyle, le myristate de méthyle, le myristate d'éthyle, le myristate de butyle, le myristate d'isobutyle, le myristate d'isopropyle, le myristate d'octyl-2 dodécyle, le monococoate d'éthyl-2 hexyle (ou monococoate d'octyle) , le palmitate de méthyle, le palmitate d'éthyle, le palmitate d'isopropyle, le palmitate d'isobutyle, le palmitate d'ethyl-2 hexyle (ou palmitate d'octyle), le stéarate de butyle, le stéarate d'isopropyle, le stéarate d'isobutyle, le stéarate d'isocétyle, l'isostéarate d'isotéaryle, , l'isostéarate d'isopropyle, le stéarate d'éthyl-2 hexyle (ou stéarate d'octyle), le pelargonate d'ethyl-2 hexyle (ou pelargonate d'octyle), l'hydroxystéarate d'éthyl-2 hexyle (ou hydroxystéarate d'octyle), le decyl oleate, l'adipate de di-isopropyle, l'adipate de di-éthyl-2 hexyle (ou adipate de di-octyle), l'adipate de diisocetyle, le succinate d'ethyl-2 hexyle (ou succinate d'octyle), le sébacate de diisopropyle, le malate d'ethyl-2 hexyle (ou malate d'octyle), le caprate/caprylate de pentaérythritol, le pentaérythritol de tétraisostéarate, l'hexanoate d'éthyl-2 hexyle (ou hexanoate d'octyle), l'octanoate d'octyldodécyle, le néopentanoate d'isodécyle, le néopentanoate d'isostéaryle, l'isononanoate de cétéaryle, l'isononanoate d'isodécyle, l'isononanoate d'isononyle, l'isononanoate d'isotridécyle, le lactate de lauryle, le lactate de myristyle, le lactate de cétyle, le propionate de myristyle, l'éthyl-2 hexanoate d'éthyl-2 hexyle (ou éthyl-2 hexanoate d'octyle), l'octanoate d'éthyl-2 hexyle (ou octanoate d'octyle, et leurs mélanges, benzoates d'alcools gras en C12-C15 (Finsolv TN de FINETEX) ; lauroyl sarcosinate d'isopropyle (Eldew SL 205 de Unipex), le dicaprylyl carbonate (Cetiol CC de Cognis).

Les huiles hydrocarbonées non volatiles peuvent être également des dialkyléthers tels que le Dicaprylyl ether (Cetiol OE de Cognis).

Les huiles hydrocarbonées non volatiles peuvent être des huiles d'origine végétale comme par exemple l'huile d'amande douce, l'huile d'avocat, l'huile de ricin, l'huile de coriandre, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore, l'huile de seigle, les triglycérides des acides caprylique/caprique comme ceux vendus par la société Stearineries Dubois ou ceux vendus sous les dénominations Miglyol 810, 812 et 818 par la société Dynamit Nobel.

Les huiles hydrocarbonées non volatiles peuvent être également choisies parmi les acides gras, les alcools gras tels que l'alcool laurique, cétylique, myristique, stéarique, palmitique, oléique ainsi que le 2-octyldodécanol.

Avantageusement, la phase huileuse comprenant au moins une huile hydrocarbonée.

Plus avantageusement, la phase huileuse comprenant au moins une huile siliconée.

De préférence, a composition cosmétique étant exempte de particules solides et de charges.

### Propulseur

Les propulseurs sont avantageusement choisis parmi le diméthyléther (DME), les hydrocarbures volatils tels que le n-butane, le propane, l'isobutane, et leurs mélanges, éventuellement avec au moins un hydrocarbure chloré et/ou fluoré. Parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT. On peut également utiliser en tant qu'agent propulseur le gaz carbonique, le protoxyde d'azote, l'azote ou l'air comprimé.

La composition contenant le ou les actifs déodorants et le ou les agents propulseurs peuvent se trouver dans le même compartiment ou dans des compartiments différents dans le récipient.

Selon l'invention, la concentration en agent propulseur varie préférentiellement entre 55% et 95% en poids par rapport au poids total de la composition pressurisée. Plus préférentiellement la concentration en agent propulseur varie entre de 70 à 85% en poids par rapport au poids total de la composition pressurisée.

On entend par « composition pressurisée », la composition totale jus+gaz contenue dans le récipient.

### Additifs

La composition peut comprendre, en outre; des diols ou les polyols comme l'éthylèneglycol, le 1,2-propylèneglycol, le 1,3-butylène glycol, l'hexylèneglycol, le diéthylèneglycol, le dipropylene glycol, le 2-éthoxyéthanol, le diéthylène glycol monométhyléther, le triéthylène glycol monométhyléther et le sorbitol. On utilisera plus particulièrement le propylèneglycol et la glycérine.

Les compositions cosmétiques selon l'invention peuvent comprendre en outre des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants, ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Le récipient peut être un récipient pressurisé, par exemple équipé d'une tige de valve creuse engagée dans un logement de la tête adapté à la recevoir.

L'invention pourra être mieux comprise à la lecture de la description détaillée qui va suivre, d'exemples de mise en œuvre non limitatifs de celle-ci, et à l'examen du dessin annexé, sur lequel :
- la figure 1 représente, de façon schématique en perspective, un exemple de tête de distribution réalisée conformément à l'invention, avant montage de la partie coopérante sur le corps de la tête,
- la figure 2 représente la tête de distribution après montage de la partie coopérante dans le corps,
- la figure 3 est une vue analogue à la figure 1 avec coupe partielle,
- les figures 4A à 4F illustrent différents agencements, parmi d'autres, de la partie coopérante et du corps,
- la figure 5 illustre la possibilité de réaliser la tête de distribution avec deux orifices de distribution selon l'invention,
- la figure 6 représente en vue de face une tête de distribution à orifices de distribution concentriques,
- la figure 7 est une coupe axiale d'une variante de réalisation de la partie coopérante,
- les figures 8A et 8B sont différents exemples de configurations de la partie coopérante de la figure 7 en vue de face, partielle,
- la figure 9 est une coupe axiale partielle d'une variante de réalisation de l'orifice de distribution,
- les figures 10A et 10B sont des vues de face selon X de différents exemples de configuration selon la figure 9,
- la figure 11 est une vue analogue à la figure 2 d'une variante de réalisation de la tête,
- les figures 12A à 12C illustrent différents exemples d'agencements des reliefs sur le corps,
- les figures 13A à 13C illustrent différents exemples de configurations de la partie coopérante par rapport au corps,
- la figure 14 est une coupe axiale partielle d'une variante de réalisation de l'orifice de distribution,
- la figure 15 est une coupe selon XV de la figure 14,
- la figure 16 est un exemple de réalisation du corps selon la figure 14, et
- la figure 17 est une vue en perspective coupée d'un exemple de tête de distribution selon la configuration de la figure 14.

Sur le dessin, les proportions respectives réelles des différents éléments constitutifs n'ont pas toujours été respectées, dans un souci de clarté.

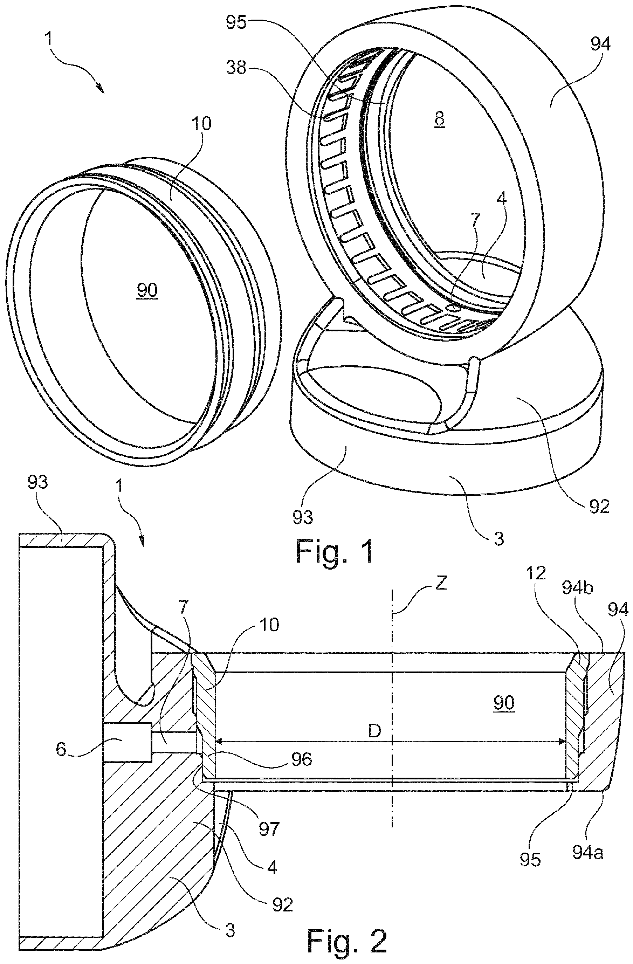

La tête de distribution 1 représentée aux figures 1 à 3 est destinée à équiper un récipient non représenté, muni d'une tige creuse de valve ou de pompe, par laquelle le produit à distribuer contenu dans le récipient est acheminé vers la tête 1.

Le récipient peut notamment être un récipient pressurisé du type bidon aérosol, contenant un gaz propulseur tel que par exemple de l'air comprimé ou un gaz liquéfié.

Le récipient peut être équipé d'une valve et l'ouverture de valve peut s'effectuer par exemple par enfoncement de la tige creuse ou en variante par basculement de celle-ci. Lorsque le récipient est équipé d'une pompe, l'actionnement de la pompe peut s'effectuer par exemple par enfoncement de la tige creuse selon son axe longitudinal.

La tête 1 comporte un corps 3, lequel peut être réalisé de façon monolithique par moulage d'une seule pièce ou comporter plusieurs éléments réalisés séparément et assemblés.

La tête de distribution 1 peut comporter, comme on peut le voir sur la figure 2, un logement 6 destiné à coopérer avec la tige creuse pour permettre au produit délivré par celle-ci de gagner un canal d'amenée 7 qui débouche dans un logement 8 du corps 3. Le logement 6 présente une dimension adaptée au diamètre extérieur de la tige, de façon à obtenir un montage étanche de la tige dans le logement 6, pour que le produit délivré par la tige passe entièrement dans le canal d'amenée 7. Ce dernier est par exemple coaxial à la tige du récipient mais pourrait être orienté autrement et comporter par exemple plusieurs portions orientées différemment.

Une partie coopérante 10, appelée noyau dans la suite lorsqu'elle est intérieure au corps, est fixée dans le logement 8 et définit par exemple avec le corps 3 un orifice de distribution 12 de section annulaire, comme illustré.

Par « section annulaire », il faut comprendre au sens de la présente invention toute section suivant un contour fermé, que ce contour soit circulaire, elliptique, polygonal ou autre.

Le noyau 10 est traversé axialement par une ouverture 90 dont le diamètre intérieur D peut être relativement important, par exemple supérieur ou égal à 10 mm, mieux 15, 20 ou 30 mm.

L'ouverture 90 contribue à conférer à la tête un aspect particulièrement esthétique. De plus, l'ouverture 90 peut permettre une circulation d'air à travers la tête sous l'effet d'entraînement d'un spray émis par l'orifice de distribution 12. Cela peut contribuer à augmenter la portée du spray et peut accroître l'effet de fraîcheur apporté par celui-ci, le cas échéant.

L'ouverture 90 peut aussi permettre d'introduire à travers la tête un doigt ou une mèche de cheveux, ce qui peut permettre d'appliquer d'un seul geste un produit sur toute la circonférence de l'élément introduit à travers la tête. Cela peut être un avantage pour appliquer par exemple un produit antiseptique ou de soin sur un doigt ou traiter une mèche de cheveux.

L'axe de distribution Z peut être perpendiculaire à l'axe longitudinal X du récipient sur lequel la tête est montée, comme illustré.

La tête 1 comporte une base 92 qui définit une surface 4 sur laquelle l'utilisateur peut appuyer pour provoquer la distribution.

La base 92 peut être prolongée inférieurement par une jupe d'habillage 93, qui recouvre la partie supérieure du récipient.

Le logement 8 qui reçoit le noyau 10 est défini par une couronne 94 d'axe Z, dont le côté inférieur rejoint la base 92. Le canal d'amenée 7 traverse la base 92 et aboutit dans le logement 8 à distance des extrémités axiales, selon l'axe Z, de la couronne 94, étant de préférence plus proche de l'extrémité arrière 94a que de l'extrémité avant 94b, comme on le voit sur la figure 2.

Le corps 3 peut présenter, comme illustré, un épaulement 95 proche de l'extrémité arrière 94a, contre lequel peut venir, le cas échéant, en butée axiale le noyau 10 au terme de son montage.

Le noyau 10 et le logement 8 peuvent présenter des surfaces annulaires 96 et 97, en contact étanche, pour fermer l'espace formé entre le noyau 10 et le corps 3 en arrière du canal d'amenée 7.

De préférence, la largeur *l* circonférentielle de l'orifice de distribution 12, autour de la direction de pulvérisation Z, est constante. On ne sort pas du cadre de la présente invention lorsque cette largeur *l* varie, par exemple de façon à tenir compte de la perte de charge éventuellement non uniforme subie par l'écoulement du produit en amont de l'orifice de distribution 12. Cette perte de charge non uniforme résulte par exemple de la géométrie de l'espace entre le noyau et le corps, notamment la présence d'angles ou de croisements. En faisant varier la largeur *l*, on peut faire en sorte que le produit puisse sortir plus facilement là où cette perte de charge est la plus grande, si l'on cherche à avoir un spray le plus homogène possible.

La largeur *l* de l'orifice de distribution est par exemple comprise entre 0,01 et 2 mm.

Le noyau 10 peut être fixé de diverses manières sur le corps 3. Dans l'exemple illustré sur les figures 1 à 3, le noyau 10 est retenu par friction sur le corps 3.

Dans l'exemple illustré, le noyau 10 est réalisé séparément du corps 3 et rapporté sur celui-ci. Le noyau 10 peut être réalisé dans la même matière thermoplastique que le corps 3 ou en variante dans une matière thermoplastique différente. On peut aussi utiliser pour réaliser le noyau 10 une matière métallique.

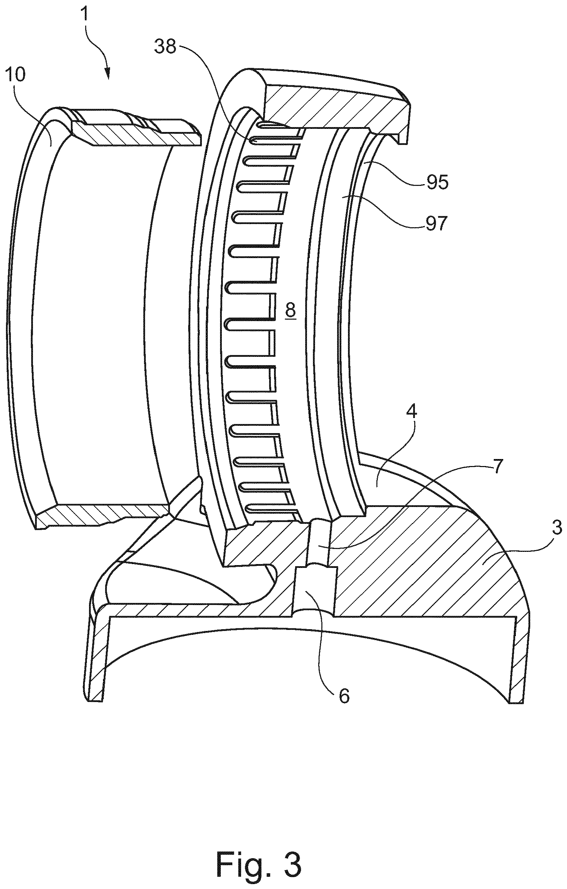

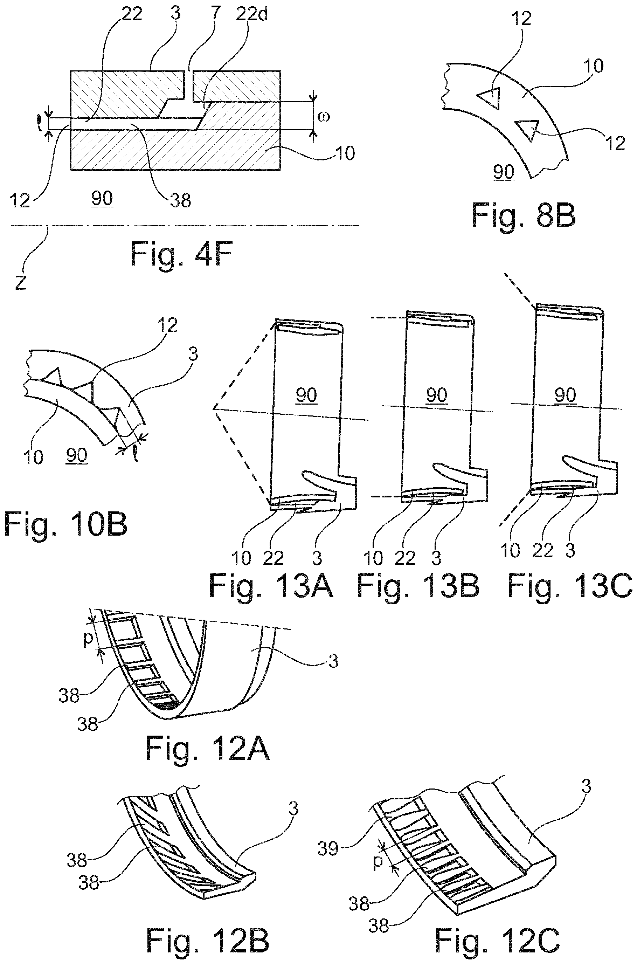

Des nervures axiales 38 sont formées sur la circonférence intérieure du logement 8, comme visible sur les figures 1 et 3 notamment, pour centrer le noyau 10 dans le logement 8. Les reliefs de centrage 38 peuvent être, comme illustré sur les figures 12A à 12C, parallèles ou obliques dans le sens circonférentiel à l'axe Z, ou courbes. Chaque relief 38 peut avoir, lorsqu'observé en vue de dessus, un contour polygonal, notamment rectangulaire ou trapézoïdal, ou en forme évasée en direction du bord de distribution. Deux reliefs de centrage 38 peuvent définir entre eux un rétrécissement 39 à proximité de l'orifice de distribution de manière à accélérer le fluide par effet venturi. Le nombre de reliefs de centrage 38 est de préférence d'au moins 10, mieux 20, encore mieux 40.

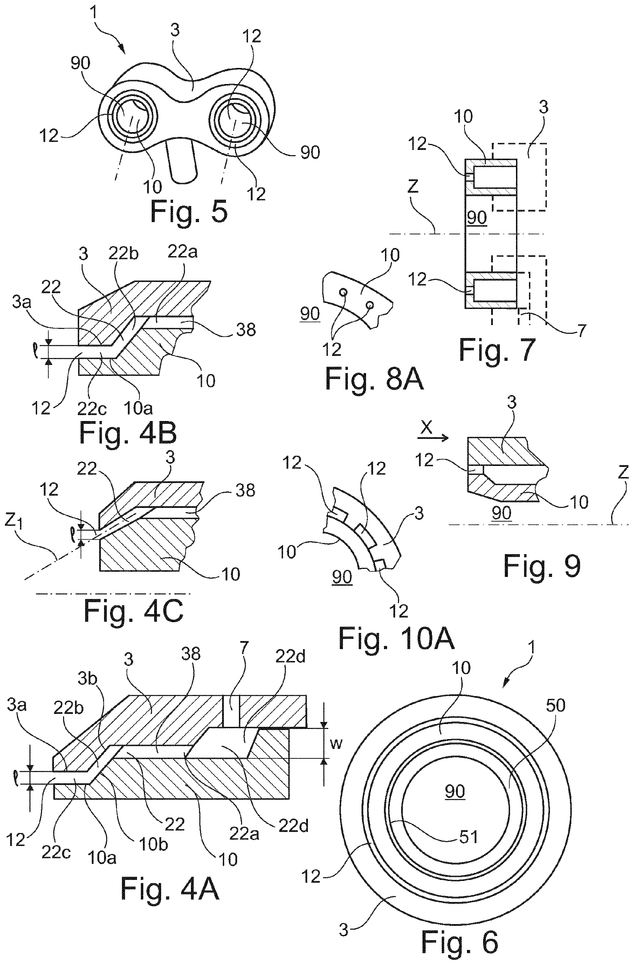

L'espace 22 formé entre le noyau 10 et le corps 3 peut présenter la configuration illustrée schématiquement à la figure 4A, et déboucher sur l'orifice de distribution 12 par une portion terminale 22c, annulaire, formée entre deux surfaces 3a et 10a, cylindriques de révolution autour de l'axe Z.

La paroi terminale 22c se raccorde à une portion proximale 22a par une portion intermédiaire inclinée 22b, formée entre des surfaces en regard 3b et 10b.

Les reliefs de centrage 38 s'étendent dans la portion proximale 22a. Cette dernière est alimentée en produit par la chambre de répartition 22d.

Lorsque l'utilisateur actionne la tête de distribution 1, le produit gagne par le canal d'amenée 7 l'espace 22 entre le noyau 10 et le corps 3 et il peut être délivré sous la forme d'un spray par l'orifice de distribution 12.

Le spray est dans l'exemple des figures 1 à 3 continu angulairement autour de l'axe de distribution, de par l'absence de contact entre le noyau 10 et le corps 3 au niveau de l'orifice de distribution 12. En effet, la ou les zones d'appui entre le noyau 10 et le corps 3 se situent par exemple, comme illustré, en retrait de l'orifice de distribution 12 d'une distance (mesurée le long de l'axe Z de distribution) d'au moins 0,5 mm.

Le spray peut être discontinu angulairement autour de l'axe de distribution, de par la présence, en particulier au niveau des reliefs 38, de contact entre le noyau 10 et le corps 3 au niveau de la sortie du produit.

De préférence, la section transversale du canal d'amenée 7 est supérieure à la section de l'orifice de distribution 12, de façon à permettre un remplissage rapide avec le produit de l'espace situé en amont de l'orifice de distribution, ce qui peut contribuer à la formation d'un spray homogène dès le début de la pulvérisation.

La chambre de répartition 22d ménagée en amont de l'espace 22a dans lequel s'étendent les reliefs de centrage 38 reçoit le produit délivré par le canal d'amenée 7.

La largeur ω de la chambre de répartition 22d est supérieure à celle / de la portion terminale 22c qui débouche sur l'orifice de distribution 12.

La chambre de répartition 22d améliore la répartition du produit avant que celui-ci ne gagne les portions plus étroites du passage par lequel s'évacue le produit.

On a illustré sur les figures 4B et 4C différents autres exemples de configurations possibles pour l'espace 22 ménagé entre le noyau 10 et le corps 3 pour la circulation du produit jusqu'à l'orifice de distribution.

Dans l'exemple de la figure 4B, l'espace 22 formé entre le noyau et le corps comporte une portion proximale 22a dans laquelle s'étendent les reliefs 38 de centrage du noyau 10 relativement au corps 3, prolongée par une portion intermédiaire 22b qui fait un angle avec la direction Z de pulvérisation, par exemple un angle rentrant. Cette portion intermédiaire 22b peut se raccorder à une portion terminale 22c, qui débouche sur l'orifice de distribution 12, cette portion terminale étant par exemple, comme illustré, définie entre deux surfaces 3a et 10a, cylindriques de révolution, parallèles à la direction de distribution Z. La variante de la figure 4B ne comporte pas de chambre de répartition.

Dans la variante de la figure 4C, la portion terminale 22c communique directement avec celle 22a dans laquelle s'étendent les reliefs de centrage 38. La portion terminale 22c fait par exemple un angle avec la direction de distribution Z. Ainsi, en demi-coupe axiale, l'axe Z₁ de l'orifice 12 est par exemple convergent, comme illustré.

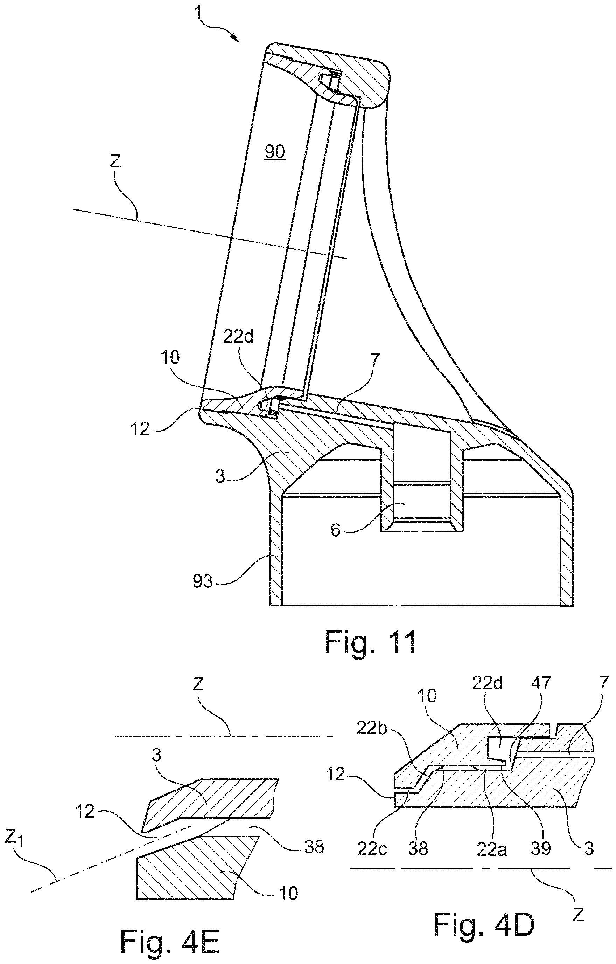

Dans la variante de la figure 4D, la partie coopérante 10 est extérieure au corps 3. La partie coopérante 10 est fixée sur le corps 3 de façon à ménager avec celui-ci la chambre de répartition 22d, en regard du canal d'amenée 7. Les portions 22a, 22b et 22c permettent d'acheminer le produit jusqu'à l'orifice de distribution 12.

Le canal d'amenée 7 débouche par exemple dans la chambre de répartition 22d par une portion orientée parallèlement à l'axe de distribution Z.

Des reliefs de centrage 38 sont réalisés par exemple sur le corps 3. La partie coopérante 10 peut être réalisée, comme illustré, avec une lèvre annulaire 39 qui délimite partiellement la chambre de répartition 22d et permet de former un rétrécissement 47 de section entre la chambre 22d et la portion 22a.

On a illustré sur la figure 4E la possibilité d'avoir un angle entre l'axe Z₂, en demi-coupe axiale, de l'orifice 12 et l'axe de distribution, qui est divergent.

Dans la variante de la figure 4F, on a illustré la possibilité de n'avoir aucun angle entre l'axe de distribution et l'axe Z de la partie coopérante 10. Le canal d'amenée 7 débouche par exemple sur une chambre de répartition 22d. Le produit est acheminé vers l'orifice de distribution 12 via des canaux 22 comportant les reliefs 38. Les reliefs 38 se prolongent jusqu'au bord de l'orifice de distribution 12 et définissent une pluralité d'orifices permettant de délivrer le produit sous la forme d'une pluralité de jets.

L'invention n'est pas limitée à une tête de distribution comportant un seul orifice de distribution 12 réalisé conformément à l'invention.

A titre d'exemple, on a illustré sur la figure 5 une tête de distribution 1 qui comporte deux orifices de distribution 12.

En présence de plusieurs orifices de distribution, ceux-ci peuvent être répartis de multiples manières sur la tête de distribution. Par exemple, les axes de pulvérisation sont parallèles, ou font un angle, étant par exemple sécants.

On a illustré aux figures 7, 8A et 8B la possibilité pour la tête de distribution d'avoir plusieurs orifices de distribution 12 formés entièrement dans le noyau 10 afin de distribuer le produit sous forme de plusieurs jets par exemple. Les orifices de distribution 12 peuvent avoir de nombreuses formes lorsqu'observés selon leur axe transversal, notamment circulaire ou triangulaire, comme illustré aux figures 8A et 8B. Les orifices de distribution 12 peuvent être percés dans le noyau 10, par exemple par perçage laser.

Le noyau 10 peut avoir en demi-section axiale, comme illustré à la figure 7, la forme d'un U. Le corps 3 peut comporter deux jupes de montage concentriques 41 qui définissent entre elles un espace de montage pour le noyau 10, et comporter en son centre une couronne 43 servant de support à la partie coopérante 10. Les jupes 41 définissent avec la couronne 43 deux canaux 45 annulaires dans lesquels se placent les pattes du U. La couronne 43 peut comporter pour chaque orifice 12 deux canaux d'amenée 22 du liquide vers cet orifice 12.

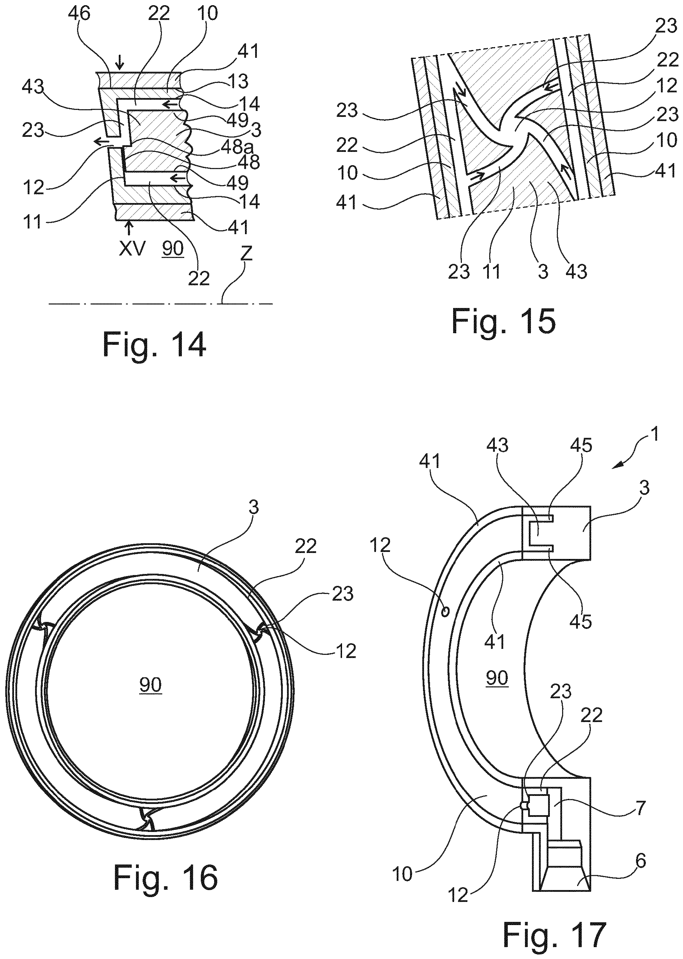

Lors du montage, comme illustré sur les figures 14 et 17, le noyau 10 peut se plaquer contre le plot 43, la face d'extrémité 48 de la couronne 43 étant en contact avec la face interne 11 du noyau 10. Les pattes du U du noyau 10 sont fixées dans les canaux 45, la face interne 46 des jupes de montage 41 étant en contact avec la face 13 du noyau 10. Les faces internes 14 des pattes du U et les surfaces latérales 49 de la couronne 43 peuvent définir entre elles les canaux d'amenée 22 du liquide vers l'orifice de distribution 12. La couronne 43 peut posséder, notamment sous forme d'empreintes, sur sa face extérieure 48, des canaux d'amenée 23 permettant le passage du liquide des canaux d'amenée 22 à l'orifice de distribution 12.

Les canaux d'amenée 22 débouchent en amont des orifices de distribution 12, sur les canaux d'amenée 23 qui mènent à l'orifice de distribution 12. Les canaux d'amenée 23 génèrent, de par leur orientation par rapport à l'orifice de distribution, un flux tourbillonnaire en sortie de l'orifice de distribution 12. Cette configuration est plus particulièrement utile dans le cas d'un gaz vecteur qui n'est pas liquéfié.

Dans une variante, les canaux d'amenée 22 peuvent être réalisés sous forme d'empreintes sur la surface latérale 49 du corps et/ou sur les faces internes 14 du noyau 10.

Dans une variante non représentée, le noyau 10 possède, notamment sous forme d'empreintes sur sa face interne 11, des canaux d'amenée 23, la face d'extrémité 48 de la couronne 43 pouvant être lisse.

Dans une variante, la couronne 43 n'est pas continue circonférentiellement et définit des plots. Les plots sont placés en amont des orifices de distribution 12 et peuvent posséder, en amont des orifices de distribution 12, les canaux d'amenée 22 et 23 tels que décrits ci-dessus.

Dans la variante des figures 4F, 9 et 10, les orifices de distribution 12 sont formés entre le noyau 10 et le corps 3, étant par exemple répartis tout autour de l'axe de pulvérisation Z. Le noyau 10 ou le corps 3 peut présenter des reliefs de centrage 38 qui délimitent circonférentiellement les orifices de distribution 12. Les reliefs de centrage 38 peuvent, comme illustré aux figures 12A à 12C, venir jusqu'au bord du noyau 10 sur toute la périphérie et définir entre eux les orifices de distribution 12. Le nombre d'orifices de distribution 12 est de préférence d'au moins 10, mieux 20, mieux encore 40. La section transversale d'un orifice de distribution 12 est par exemple supérieure à 0,003 mm². Les orifices de distribution 12 sont de préférence espacés d'un espace d'au moins 1 mm, qui est le même que le pas p entre les reliefs de centrage. Comme illustré aux figures 10A et 10B, les orifices de distribution 12 peuvent être de section transversale polygonale, notamment triangulaire.

Le noyau 10 peut s'étendre, comme illustré à la figure 13A, en retrait par rapport au corps d'une valeur comprise entre 0 et 1 mm, mieux entre 0 et 0,5 mm. Le corps 3 déborde de l'orifice de distribution et peut générer un spray convergent.

Le noyau 10 peut être, comme illustré à la figure 13B, affleurant au corps 3. Le spray peut alors être droit.

Le noyau 10 peut s'étendre, comme illustré à la figure 13C, en avant par rapport au corps 3 d'une valeur comprise entre 0 et 1 mm, mieux entre 0 et 0,5 mm. Le spray peut alors être divergent.

On ne sort pas du cadre de la présente invention lorsqu'un orifice de distribution supplémentaire est présent, par exemple en rapportant à l'intérieur du noyau 10 un deuxième noyau 50 qui définit avec le premier noyau 10 un deuxième orifice de distribution 51, coaxial au premier, comme illustré à la figure 6. Un passage 90 reste ménagé à travers la tête de distribution.

L'orifice de distribution peut être alimenté par plus d'un produit.

La tête de distribution peut être alimentée par deux produits qui sont distribués par des orifices de distribution distincts.

L'axe Z peut être non perpendiculaire à l'axe de la tige du récipient sur lequel la tête est montée, comme illustré à la figure 11. Dans cet exemple, l'axe Z est orienté vers le haut lorsque le récipient est vertical avec la tête de distribution en haut.

Le canal d'amenée 7 peut être orienté sensiblement parallèlement à l'axe de distribution Z, au moins pour la portion qui débouche en regard de la partie coopérante 10. Cette dernière peut être réalisée avec une lèvre annulaire 39 qui définit un rétrécissement de section 47.

La configuration peut être similaire à celle de la figure 4D hormis le fait que la partie coopérante 10 est extérieure au corps 3 dans l'exemple de la figure 4D et intérieure dans l'exemple de la figure 11.

La tête de distribution peut être agencée pour permettre le montage d'un capot de protection et comporter, le cas échéant, un système on/off permettant d'interdire l'actionnement du dispositif pour une certaine position de la tête de distribution par rapport au récipient ou pour une certaine position d'un élément de verrouillage de la tête de distribution relativement à celle-ci.

Dans des variantes non illustrées, l'orifice de distribution est formé entre un corps et une partie coopérante, le corps étant radialement intérieur par rapport à la partie coopérante, le canal d'amenée du produit traversant le corps. Toutes les caractéristiques décrites en référence aux figures peuvent se retrouver dans de telles variantes où le corps est radialement intérieur par rapport à la partie coopérante.

L'expression « comportant un » doit se comprendre comme étant synonyme de « comportant au moins un ».

### Exemple :

### i) Dispositif

On prépare trois dispositifs équipés d'une tête de distribution représentée sur la figure 1. On vait varier :
- le nombre de trous et la section des trous de la tête de distribution, et
- les compositions chimiques contenues dans ces dispositifs.

### ii) Composition chimique

On évalue les compositions commerciales suivantes :
Dispositif 1 : AEROSOL AT Clear femmes Bi-O invisible ® Garnier
Dispositif 2 : AEROSOL AT GARNIER MINERAL Invisi Dry Control®
Dispositif 3 : AEROSOL ANTITRANSPIRANT MINERAL ULTRA® Garnier

### iii) Evaluation des dispositifs

### a) Evaluation de la qualité du spray

| Numéro de dispositif | Nb de trou | Section trou en mm² | Pulvérisation sans bouchage |
|---|---|---|---|
| Dispositif 1 | 5 | 0.0452 | +++++++++++++ |
| | | | |
| Dispositif 2 | 5 | 0.0452 | - |
| | 6 | 0.16 | +++++++++++++ |
| | | | |
| Dispositif 3 | 5 | 0.0452 | +++++++++++++ |
| | 6 | 0.16 | +++++++++++++ |

| | | | |
|---|---|---|---|
| +++++++++++++ : très bon visuel sans aucun bouchage - : bouchage | | | |

### b) Conclusion du tableau précédent

Les dispositifs 1, 2 et 3 conduisent tous à une très bonne qualité de spray pour une section de trou supérieure à 0.05 mm².

## Revendications

1. Récipient équipé d'une tête de distribution, le récipient contenant une composition cosmétique à distribuer, **caractérisé par le fait que** :
ii. la tête de distribution comporte :
- un corps (3) comportant une couronne (94) s'étendant selon un axe de distribution (Z), le corps étant ouvert à ses deux extrémités axiales opposées,
- une partie coopérante (10) s'étendant selon un axe de distribution (Z) et étant ouverte à ses deux extrémités axiales opposées, définissant au moins partiellement au moins un orifice de distribution (12), la partie coopérante (10) étant engagée sur la couronne (94), la section transversale de l'orifice de distribution étant comprise entre 0,05 mm² et 0,5 mm²,
- ii. la composition cosmétique comprend, dans un milieu cosmétiquement acceptable,
- au moins une phase huileuse comprenant une ou plusieurs huiles choisie(s) parmi les huiles siliconées, hydrocarbonées et fluorées,
- au moins un actif cosmétique, notamment au moins un actif déodorant et/ou un actif anti-transpirant.

2. Récipient selon la revendication 1, l'orifice de distribution (12) étant annulaire et présentant de préférence, dans le sens circonférentiel, une largeur (*l*) constante.

3. Récipient selon l'une quelconque des revendications précédentes, le ou les orifice(s) de distribution (12) étant à symétrie axiale, de préférence à symétrie de révolution.

4. Récipient selon l'une quelconque des revendications précédentes, l'orifice de distribution (12) étant de section transversale triangulaire.

5. Récipient selon la revendication 1, la partie coopérante (10) définissant au moins partiellement une pluralité d'orifices de distribution (12), la somme des sections transversales des orifices de distribution étant comprise entre 0,05 mm² et 0,5 mm²,

6. Récipient selon la revendication 4, le nombre d'orifices de distribution (12) étant supérieur ou égal à 5, de préférence supérieur ou égal à 10.

7. Récipient selon l'une quelconque des précédentes, la section transversale des orifices de distribution étant comprise entre 0,03 mm² et 0,4 mm², et de préférence entre 0,05 mm² et 0,4 mm².

8. Récipient selon l'une quelconque des revendications précédentes, la phase huileuse comprenant au moins une huile hydrocarbonée.

9. Récipient selon l'une quelconque des revendications précédentes, la phase huileuse comprenant au moins une huile siliconée.

10. Récipient selon l'une quelconque des revendications précédentes, la composition cosmétique étant exempte de particules solides et de charges.

11. Procédé cosmétique comprenant la mise en œuvre du récipient selon l'une quelconque des revendications 1 à 10.

## Patentansprüche

1. Behälter, der mit einem Ausgabekopf ausgestattet ist, wobei der Behälter eine auszugebende kosmetische Zusammensetzung enthält, **dadurch gekennzeichnet, dass**:
ii. der Ausgabekopf beinhaltet:
- einen Körper (3), der einen Kranz (94) beinhaltet, der sich entlang einer Ausgabeachse (Z) erstreckt, wobei der Körper an seinen beiden entgegengesetzten axialen Enden offen ist,
- einen kooperierenden Teil (10) der sich entlang einer Ausgabeachse (Z) erstreckt und an seinen beiden axial entgegengesetzten Enden offen ist, wobei er mindestens teilweise mindestens eine Ausgabeöffnung (12) definiert, wobei der kooperierende Teil (10) in den Kranz (94) eingesetzt ist, wobei der Querschnitt der Ausgabeöffnung zwischen 0,05 mm² und 0,5 mm² beträgt,
- ii. die kosmetische Zusammensetzung in einem kosmetisch akzeptablen Medium umfasst:
- mindestens eine Ölphase, die ein oder mehrere Öle umfasst, das(die) unter Silikonölen, Kohlenwasserstoffölen und fluorierten Ölen gewählt ist(sind),
- mindestens einen kosmetischen Wirkstoff, insbesondere mindestens einen desodorierenden Wirkstoff und/oder einen schweißhemmenden Wirkstoff.

2. Behälter nach Anspruch 1, wobei die Ausgabeöffnung (12) ringförmig ist und bevorzugt in Umfangsrichtung eine konstante Breite (*l*) aufweist.

3. Behälter nach einem der vorhergehenden Ansprüche, wobei die Ausgabeöffnung(en) (12) axialsymmetrisch, bevorzugt rotationssymmetrisch ist(sind).

4. Behälter nach einem der vorhergehenden Ansprüche, wobei die Ausgabeöffnung (12) einen dreieckigen Querschnitt hat.

5. Behälter nach Anspruch 1, wobei der kooperierende Teil (10) mindestens teilweise eine Mehrzahl von Ausgabeöffnungen (12) definiert, wobei die Summe der Querschnitte der Ausgabeöffnungen zwischen 0,05 mm² und 0,5 mm² beträgt.

6. Behälter nach Anspruch 4, wobei die Anzahl der Ausgabeöffnungen (12) größer oder gleich 5, bevorzugt größer oder gleich 10 ist.

7. Behälter nach einem der vorhergehenden Ansprüche, wobei der Querschnitt der Ausgabeöffnungen zwischen 0,03 mm² und 0,4 mm² und bevorzugt zwischen 0,05 mm² und 0,4 mm² beträgt.

8. Behälter nach einem der vorhergehenden Ansprüche, wobei die Ölphase mindestens ein Kohlenwasserstofföl umfasst.

9. Behälter nach einem der vorhergehenden Ansprüche, wobei die Ölphase mindestens ein Silikonöl umfasst.

10. Behälter nach einem der vorhergehenden Ansprüche, wobei die kosmetische Zusammensetzung frei von Feststoffpartikeln und Füllstoffen ist.

11. Kosmetisches Verfahren, das den Einsatz des Behälters nach einem der Ansprüche 1 bis 10 umfasst.

## Claims

1. Receptacle equipped with a dispensing head, the receptacle containing a cosmetic composition to be dispensed, **characterized in that**:
i. the dispensing head comprises:
- a body (3) comprising a ring (94) extending along a dispensing axis (Z), the body being open at its two opposite axial ends,
- an interacting part (10) extending along a dispensing axis (Z) and being open at its two opposite axial ends, defining at least partially at least one dispensing orifice (12), the interacting part (10) being engaged on the ring (94), the transverse section of the dispensing orifice being between 0.05 mm² and 0.5 mm²,
- ii. the cosmetic composition comprises, in a cosmetically acceptable medium,
- at least one oily phase comprising one or more oils chosen from silicone, hydrocarbon and fluorinated oils,
- at least one cosmetic active ingredient, in particular at least one deodorant active ingredient and/or one antiperspirant active ingredient.

2. Receptacle according to Claim 1, the dispensing orifice (12) being annular and preferably having a constant width (1) in the circumferential direction.

3. Receptacle according to either of the preceding claims, the one or more dispensing orifices (12) having axial symmetry, preferably having rotational symmetry.

4. Receptacle according to any one of the preceding claims, the dispensing orifice (12) having a triangular transverse section.

5. Receptacle according to Claim 1, the interacting part (10) defining at least partially a plurality of dispensing orifices (12), the sum of the transverse sections of the dispensing orifices being between 0.05 mm² and 0.5 mm².

6. Receptacle according to Claim 4, the number of dispensing orifices (12) being greater than or equal to 5, preferably greater than or equal to 10.

7. Receptacle according to any one of the preceding claims, the transverse section of the dispensing orifices being between 0.03 mm² and 0.4 mm², and preferably between 0.05 mm² and 0.4 mm².

8. Receptacle according to any one of the preceding claims, the oily phase comprising at least one hydrocarbon oil.

9. Receptacle according to any one of the preceding claims, the oily phase comprising at least one silicone oil.

10. Receptacle according to any one of the preceding claims, the cosmetic composition being free from solid particles and fillers.

11. Cosmetic method comprising the implementation of the receptacle according to any one of Claims 1 to 10.
